# EUROPEAN PATENT SPECIFICATION

(11) **EP 0 956 244 B1**
(45) Date of publication and mention of the grant of the patent: **17.09.2003**
(21) Application number: 98903495.4
(22) Date of filing: 15.01.1998
(51) Int. Cl.: B65D 71/12

(54) **ARTICLE CARRIER AND BLANK THEREFOR**
ARTIKELTRÄGER UND ZUSCHNITT DAFÜR
PORTE-BOUTEILLES OU AUTRE ET EBAUCHE ASSOCIEE

(30) Priority: 16.01.1997 GB 9700832
(43) Date of publication of application: 17.11.1999
(73) Proprietor: THE MEAD CORPORATION, Dayton Ohio 45463 (US)
(72) Inventor: BUCK, Simon, Yate Bristol BS37 7FB (GB)
(74) Representative: Hepworth, John Malcolm
(86) International application number: US9800723
(87) International publication number: WO98031603

(56) References cited:
- EP-A- 0 171 229
- WO-A-98/10998
- DE-A- 2 359 805
- US-A- 3 011 677
- US-A- 3 543 472
- US-A- 3 570 706
- US-A- 4 509 640
- US-A- 5 520 283

## Description

This invention relates to an article carrier according to the preamble of claim 1 of the basket type adapted to accommodate a plurality of articles, such as bottles and to a blank according to the preamble of claim 6 for forming the carrier as well as to a method of loading such article carriers. Normally a basket carrier for bottles includes a central (medial) partition structure which incorporates a handle structure by which the carrier can be lifted and carried and the bottles are arranged in rows on either side of the partition structure. More often than not, bottles are separated from one another by transverse partition panels extending from each side of the medial partition structure to the adjacent side wall of the carrier. Hence, in this type of arrangement the bottles are accommodated in individual cells of the carrier although such cells are not essential.

Known basket carriers require a series of complex folds to form partition structures, which may require the blank to be rotated or inverted during construction, thus slowing the process down. The present invention overcomes this problem by constructing a carton which adopts a difference approach to supporting and separating articles in a carton.

One example of a basket style carrier is illustrated in US 3 570 706 which discloses a carrier having side and end walls, a bottle partition structure and bottom wall with sloping panel portions provided with bottle apertures. The bottle apertures have overlying tabs to protect part of the bottle in the cut out.

DE 2 359 805 (corresponding to the preambles of claims 1 and 6) discloses another example of basket style carrier having side and end walls and a bottom wall structure. There further comprises a partition structure struck from and connected to one side of the side and end walls and a plurality of tabs struck from and hingedly connected to base wall structure. In use, the tabs form article cells to space adjacent articles held in the carrier.

EPO171229B1 discloses a known bottle carrier of the wraparound type having top, bottom and side walls interconnected to form a tubular open-ended structure. A sloping heel panel interconnects each side wall along a fold line with an associated bottom lap panel. The bottom corners of the carton formed by the sloping heel panel comprise bottle heel receiving apertures formed in each sloping heel panel which extend into the associated side wall and lap panels. The receiving apertures form part of the bottle heel retaining feature which further comprises bottle engaging and carrier reinforcing flaps which are foldably joined along opposed peripheral edges of the bottle heel receiving apertures. In particular, EPO171229B1 discloses bottle engaging and carrier reinforcing flaps which extend across the associated sloping heel panel and into the associated side wall and lap panel; the flaps comprising hinged portions so that in the formed bottle carrier a portion ofthe bottle engaging and carrier reinforcing flap is substantially parallel with the inner surface of the associated lap panel. In this construction a portion of the heel of a bottle protrudes from the carrier without being protected and the tighter the package is made the more is the tendency for the heel to protrude further. Also, tears can be created in the carton surrounding a receiving aperture due sometimes to an "over-tight" package and in part at least, to any movement of a bottle acting directly against the edge of the receiving aperture.

Another example of a bottle heel retaining structure is shown in US 5 520 283 which discloses a wrap around carrier with a bottle receiving aperture struck from the side panel and intermediate panel and corresponding receiving flaps folded inwardly to receive a portion of a bottle.

The present invention adopts a bottle heel retention means to support articles in a basket type carrier. Thus, the need for complex partition structures is reduced, with the consequent savings in paperboard.

Basket type carriers differ from wraparound carriers in that they are pre-formed cartons which simply need to be set up from a collapsed condition ready for loading. A problem with adapting bottle keel retention means in a basket type carrier is that known folding methods cannot be applied to a carrier with fixed side and end wall panels because the articles can interfere with folding for example. The invention seeks to solve or at least mitigate this problem. A carton of the present invention can be formed in a straight line gluing machine. It is envisaged that the invention can be used in an adapted wraparound machine, thus removing the need for dedicated machinery.

One aspect of the invention provides an article carrier of the basket type adapted to accommodate a plurality of articles, for example, bottles, formed from a blank comprising, in sequence, a first side panel, a first end panel, a second side panel and a second end panel hinged one to the next. The carrier further comprises an internal partition structure handle means and article support and separating means. The base comprises a pair of base panels each is associated with respective ones of the side walls and an intermediate panel is hinged between each base panel and the associated respective ones of the side walls. The article support and separating means is struck from respective ones of intermediate panels and base panels, and comprises an aperture adapted to receive a portion of an article in use and a flap struck from and hingedly connected to an edge of the aperture. The flap is adapted to pivot inwardly of a carrier formed from the blank about its hinged connection.

Preferably, each of said article support and separating means comprises a pair of complementary hinged flaps, each being hinged along opposite edges of the aperture.

According to an optional feature of this aspect of the invention, the internal partition structure comprises a plurality of integral transverse partition panels wherein the transverse partition panels interconnect the handle means to each of the opposed side walls of the carrier, thereby creating a plurality of article receiving cells on both sides of the handle means.

According to another optional feature of this aspect of the invention a tab extends from each of the side panels into each of the apertures, each tab being interrupted by substantially vertical cut line extending from a central portion of the lower edge of the tab and wherein the tab is disposed so as to abut and support a portion of an article located at least in part in the aperture.

In some embodiments, each of the flaps includes an upper portion and a lower portion hinged so as to be foldable relative to one another and thereby allow the flap to bend and conform to the shape of an adjacent portion of an article and wherein the hinged connection between the upper and lower flap portions is disposed above the base of an article engaged therewith.

According to a second aspect of the invention there is provided a blank for forming an article carrier of the basket type comprises in sequence a first side panel, a first end panel, a second side panel and a second end panel, hinged one to the next, first and second base panels hinged to respective ones of the first and second intermediate panels which are in turn hingeably connected to the first and second side panels respectively and article support and separating means. The article support and separating means is struck from respective ones of intermediate panels and base panels, and comprises an aperture adapted to receive a portion of an article in use and a flap struck from and hingedly connected to an edge of the aperture, the flap is adapted to pivot inwardly of a carrier formed from the blank about its hinged connection.

Preferably there further comprises first and second handle panels being connected to respective ones of said end panels, and wherein a plurality of transverse partition panels interconnect the handle panels to each of the opposed side panels of the carrier, thereby creating a plurality of article receiving cells on both sides of the handle means when a carrier is formed from the blank.

A further aspect of the invention provides a method of loading a basket carrier, which method comprises the steps:
(i) setting up the carrier with its base panels open;
(ii) folding intermediate and base panels outwardly out of alignment with respective side panels;
(iii) introducing articles to the carton;
(iv) pivoting flaps of the article securing and supporting means about their hingeable connections into operative positions;
(v) folding the intermediate and base panels inwardly around the articles such that the flaps receive and support respective articles;
(vi) interengaging the base panels.

Embodiments of the invention will now be described, by way of example, with reference to the accompanying drawings, in which:-
FIGURE 1 is a plan view of an unfolded single blank of paperboard from which a basket carrier according to the invention is formed;
FIGURE 2 is a perspective view of an erected basket carrier formed from the blank shown in Figure 1 viewed from above and from one end;
FIGURE 4 is a perspective view of a part erected basket carrier formed from the blank shown in Figure 1 viewed from above and from one end.

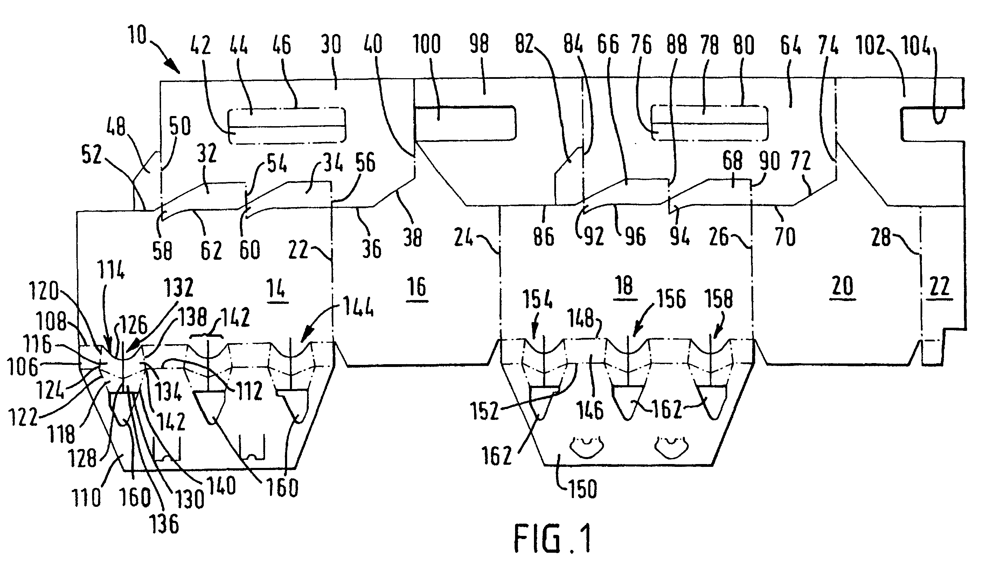

Referring to the drawings, and in particular to Figure 1 thereof, a bottle carrier is formed from a single blank 10 of paperboard or other suitable foldable sheet material and is adapted to accommodate six bottles arranged in two rows of three bottles each. The blank includes first side panel 14, first end panel 16, second side panel 18 and second end panel 20 and glue flap 22 hingeably connected one to the next along fold lines 22,24,26 and 28 respectively.

An outer handle panel 30 is disposed adjacent first side panel 14 and first end panel 16. Handle panel 30 is separated from side panel 14 by transverse partition panels 32, 34, (described in more detail below) and from end panel 18 by cut lines 36, 38. Handle panel 30 is hingeably connected to first end panel 16 along fold line 40 which forms part of one side edge of handle panel 30. Cut lines 36, 38 extend between fold lines 22 and 40. Outer handle panel 30 includes a hand aperture 42. A hand cushioning flap 44 is connected along fold lines 48 to an upper edge of hand aperture 42. A medial support tab 48 is hingeably connected to the opposing side edge of handle panel 30 along fold line 50, as shown in Figure 1. Tab 48 is adjacent to side wall 14 but separated from it by cut line 52.

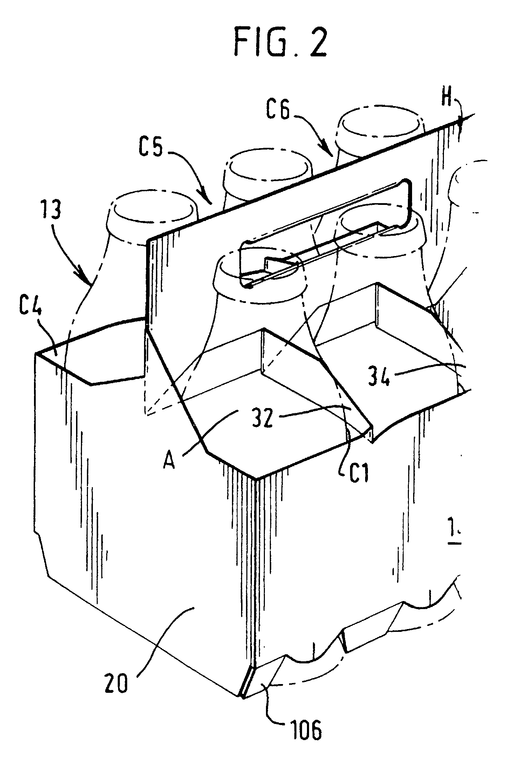

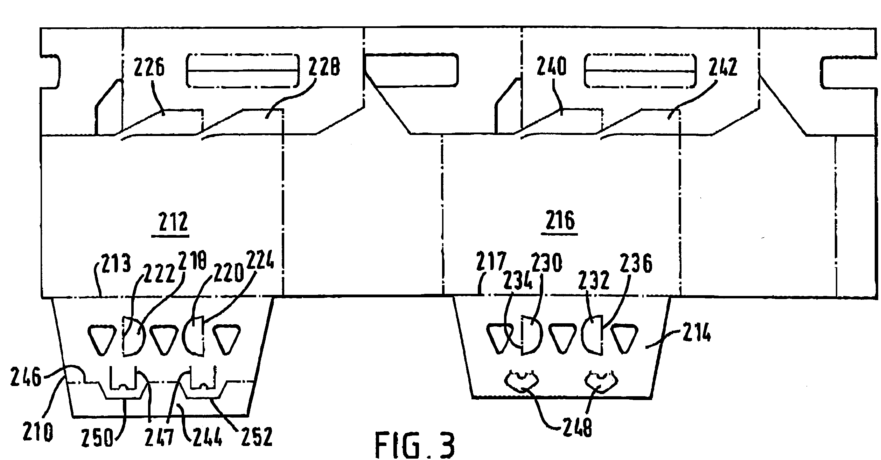

As shown in Figures 1 and 2, one side of a partition structure 12 for the article carrier is formed from the lower portion of the outer handle panel 30 and side panel 14 and comprises a pair of transverse partition panels 32, 34. Transverse partition panel 32 is connected to handle panel 30 along fold line 54. Likewise, transverse partition panel 34 is connected to handle panel 30 along fold line 56. Connecting portions 58, 60 extend between and interconnect respective ones of transverse partition panels 32, 34 and first side panel 14.

Transverse partition panel 32 is separated from first side panel 14 by cut line 62 which extends from fold line 54 and terminates short of cut line 52 to define the lower edge of transverse partition panel 32. The upper edge of transverse partition panel 32 is defined by an extension of cut line 52 which extends to fold line 54.

Likewise, transverse partition panel 34 is separated from first side panel 14 by cut line 36 which extends from fold line 56 and terminates short of cut line 62 to define the lower edge of transverse partition panel 34. The upper edge of transverse partition panel 34 is defined by an extension of cut line 62 which extends to fold line 56.

An outer handle panel 64 is disposed adjacent second side panel 18 and second end panel 20. Handle panel 64 is separated from side panel 18 by transverse partition panels 66, 68 (described in more detail below) and from end panel 20 by cut lines 70, 72. Handle panel 64 is hingeably connected to second end panel 20 along fold line 74 which forms part of one side edge of handle panel 64. Cut lines 70, 72 extend between fold lines 26 and 74. Outer handle panel 64 includes a hand aperture 76. A hand cushioning flap 78 is connected along fold lines 80 to an upper edge of hand aperture 76. A medial support tab 82 is hingeably connected to the opposing side edge of handle panel 64 along fold line 84, as shown in Figure 1. Tab 82 is adjacent to side wall 14 but separated from it by cut line 86.

As shown in Figures 1 and 2, the other side of partition structure 12 for the article carrier is formed from the lower portion of the outer handle panel 64 and side panel 18 and comprises a pair of transverse partition panels 66, 68. Transverse partition panel 66 is connected to handle panel 64 along fold line 88. Likewise, transverse partition panel 68 is connected to handle panel 64 along fold line 90. Connecting portions 92, 94 extend between and interconnect respective ones of transverse partition panels 66, 68 and first side panel 18.

Transverse partition panel 66 is separated from second side panel 18 by cut line 96 which extends from fold line 88 and terminates short of cut line 86 to define the lower edge of transverse partition panel 66. The upper edge of transverse partition panel 66 is defined by an extension of cut line 86 which extends to fold line 88.

Likewise, transverse partition panel 68 is separated from second side panel 18 by cut line 70 which extends from fold line 90 and terminates short of cut line 96 to define the lower edge of transverse partition panel 68. The upper edge of transverse partition panel 68 is defined by an extension of cut line 96 which extends to fold line 90.

An inner handle panel 98 is disposed adjacent part of first end panel 16 and part of second side panel 18. Handle panel 98 is separated from end panel 16 and side panel 18 by an extension of cut line 86. Handle panel 98 is hingeably connected to a side edge of outer handle panel 64 along fold line 84. Inner handle panel 98 includes a hand aperture 100 positioned intermediate upper and lower edges of inner handle panel 98. A second inner handle panel 102 is disposed adjacent second end panel 20 and glue flap 22. Handle panel 102 is separated from end panel 20 and glue flap 22 by a cut line 104. Handle 102 is hingeably connected to the opposing side edge of outer handle panel 64 along fold line 74. Inner handle panel 102 includes a hand aperture 104 positioned intermediate upper and lower edges of inner handle panel 102.

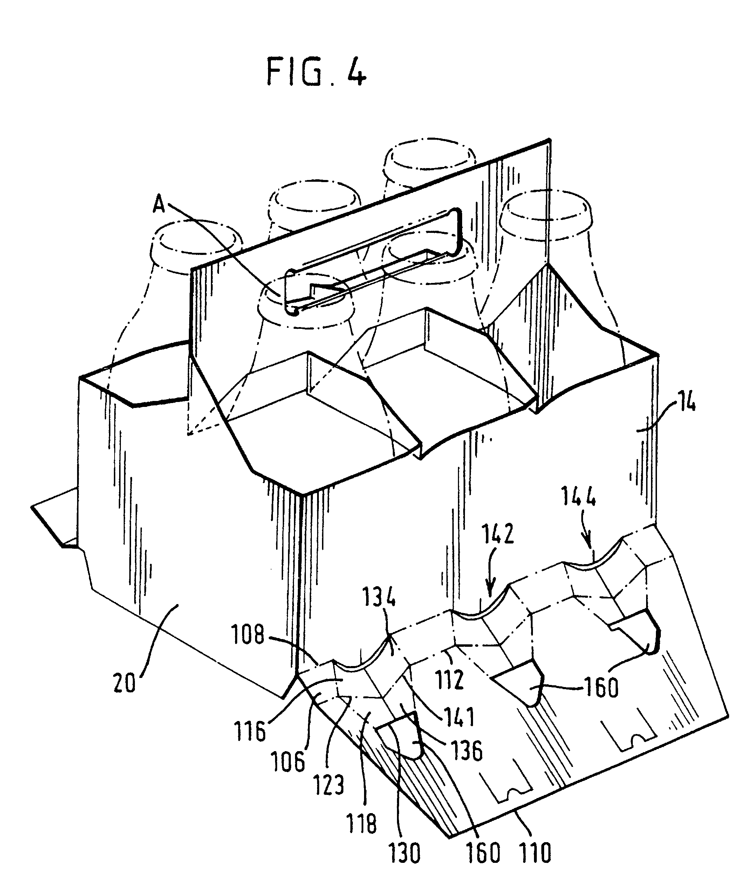

Turning to the construction of the base, on one side of the partition structure 12 illustrated in Figures 1 and 4, there is an intermediate panel 106 hingeably connected to side wall 14 along interrupted fold line 108 and to a base panel 110 along fold line 112. The intermediate panel 106 is interrupted by article support and separating means which comprises a first flap 114 comprising upper and lower flap portions 116, 118. Upper portion 116 is hinged to intermediate panel 106 along fold line 120. Lower portion 118 is hinged to base panel 110 along fold line 122 and to upper portion along fold line 123. A cut line 124 extends from fold line 120 to fold line 122 to bi-sect fold line 112. These fold lines 120, 122 and cut line 124 together define a lateral edge of first flap 114 as shown in Figure 1. First flap 114 is struck from intermediate panel 106 and base panel 110 by an upper arcuate cut line 126, central cut line 128 and bottom cut line 130 and is pivotal about hinged connections 120, 122.

Similarly, a complementary second flap 132 comprises upper and lower flap portions 134, 136 respectively. Thus, upper portion 134 is hinged to intermediate panel 106 along fold line 138. Lower portion 136 is hinged to base panel 110 along fold line 140 and to upper portion along fold line 141. A cut line 142 extends from fold line 138 to fold line 140 to bi-sect fold line 112. These fold lines 138, 140 and cut line 142 together define a lateral edge of second flap 132 as shown in Figure 1. Second flap 132 is struck from carton panels 106 and 110 by an extension of upper cut line 126, central cut line 128 and an extension of bottom cut line 130 and is pivotal about hinged connections 138, 140.

In this embodiment of the invention, second flap 132 is a mirror image of first flap 114 along the central cut line 128. They need not necessarily be mirror images, and indeed only one flap might be provided in a given article and support separation means. Also it is envisaged that where two flaps are provided, they need not abut along cut line 128, and indeed a significant gap could be provided between the flaps for example.

Arcuate cut line 126 also defines the lower edge of an extended portion of side panel 14. It is interrupted in a central portion by an extension of cut line 128 extending into side panel 14. In use, this portion of the carrier is provided to support an article and to provide some flexibility to the side panel 14.

In the embodiment shown in Figure 1, there are three pairs of flaps 114, 132; 142, 144 struck from the intermediate panel 106 and base panel 112 to receive three articles A. The position of each pair of flaps 114, 132, 142, 144 is in alignment with each article receiving cell C, shown in Figure 2 so that in use these flaps are able to separate and support an article in each cell.

Likewise, on the other side of the partition structure 13 illustrated in Figures 1 and 2, there is a second intermediate panel 146 struck from side panel 18 along interrupted fold line 148 and to a second base panel 150 along fold line 152. The intermediate panel 146 is interrupted by article support and separating means being substantially the same as the article support and separating means formed from panels 14, 106 and 110 (described above) and is therefore not described in any greater detail. Thus, three pairs of flaps 154, 156, 158 are struck from the intermediate panel 146 and base panel 150 to receive three articles A. The position of each pair of flaps 154, 156, 158 is in alignment with each article receiving cell C.

Optionally, three apertures 160 are struck from base panel 110, each aperture extending from the lower edges of respective ones of pairs of flaps 114, 132, 142, 144. Likewise, three apertures 162 are struck from base panel 150, each aperture extending from the lower edges of respective ones of pairs of flaps 154, 156, 157. In use, these apertures 160, 162 are used by packaging machinery to assist in forming the lower panels of the carton around the articles.

Preferably, a pair of generally rectangular locking tabs 164 are struck from base panel 110 and a complementary pair of locking tabs 166 are struck from second base panel 150. It will be understood by those skilled in the art that other methods of interlocking the base panels together during construction can be adopted and the invention is not limited to locking tabs illustrated in the embodiments.

The construction of a completed carrier of the first embodiment shown in Figures 1, 2 and 4 in a flat collapsed condition from the blank requires a series of sequential folding and gluing operations which can be performed in a straight line gluing machine so that the carton is not required to be rotated or inverted to complete its construction. The folding process is not limited to that described below and can be altered according to particular manufacturing requirements.

Thus, inner handle panels 98, 102 are folded about fold lines 84 and 74 respectively shown in Figure 1 and secured into a face to face relationship with outer handle panel 64. The handle panels 64, 98, 102 may be connected together by glue or other means known in the art. Glue flap 22 is folded about fold line 28 into a face to face relationship with second end panel 20.

Second end panel 20, second side panel 18 and outer handle panel 64 are folded about fold line 24 such that they form a face to face relationship with first end wall 16, second side panel 14 and first outer panel 30 respectively. By folding the panels in this way, the two sides of the basket carrier are brought into a face to face relationship with each other and inner handle panels 98, 102 are secured together with the inner face of outer handle panel 30 by glue or other means known in the art. In addition, glue flap 22 is secured to first side panel 14 and medial support tabs 48 and 82 are secured to the upper portion of respective ones of end panels 20 and 16 respectively by glue or other means known in the art.

The carton is then at an intermediate stage: a completed collapsed article carrier.

In use, the article carrier is erected by moving the outer edges of the collapsed carrier defined by fold lines 24 and 28 inwardly towards each other. This moves end panels 16 and 20, and side panels 14 and 18 into a rectangular configuration. This action facilitates the construction of individual cells. Thus, transverse partition panels 32, 34 are moved out of alignment with first side panel 14 and handle panel 30 and are folded about fold lines 54 and 56 respectively such that transverse partition panels 32, 34 are in a substantially perpendicular relationship with both end panel 30 and side panel 14. As illustrated in Figure 2, three cells C1, C2, C3 are thus formed. Similarly, transverse partition panels 66, 68 are moved out of alignment with second side panel 18 and handle panel 64 and are folded about fold lines 88, 90 respectively so that transverse partition panel 66, 68 are in a perpendicular relationship to both handle panel 64 and side wall panel 18, so that three further cells C4, C5, C6 are formed. In use, the transverse partition panels may separate and support the upper portions of articles in adjacent cells.

The carton is then ready to receive articles which are loaded by relative vertical movement between the bottles and carrier during forward feed movement well known in the art by the which the bottles enter their respective cells through the open bottom of the carrier. Alternatively, the bottles can enter their respective cells through the top of the carrier.

Thereafter, the base is formed which in the first embodiment requires intermediate 106 and base panels 110 to be folded out of alignment from side panel 14 and away from the article receiving cells along fold line 108 illustrated in Figure 4. Likewise, intermediate 146 and base panels 150 are folded out of alignment from side panel 18 and away from article receiving cells along fold line 148. The article support and separating means is then constructed. Flap 114 is displaced inwardly by suitable means thereby causing upper and lower flap portions 116, 118 to be folded about fold lines 120, 122 respectively into a juxtaposed relationship with intermediate panel 106 and base panel 110 respectively. Likewise, flap 132 is displaced inwardly by suitable means thereby causing upper and lower flap portions 134, 136 to be folded about fold lines 138, 140 respectively into a juxtaposed relationship with intermediate panels 106 and base panel 110 respectively. The arcuate cut line 126 is shaped so that the folding of flaps 114 and 132 is not interfered with by the article A, shown in Figure 4. The other flaps 142, 144, 154, 156, 158 are folded in substantially the same way as flaps 114 and 132 and are therefore not described in any greater detail. It will be understood by those skilled in the art that the flaps can be folded at approximately the same time, if desired.

Intermediate panels 112, 146 are then folded inwardly about fold lines 108, 148 and base panels 110, 150 are folded inwardly about 112, 152 respectively. Thus, flaps 114, 132; 142; 144; 154; 156; 158 are brought into contact with respective ones of articles A such that the upper and lower portions of each flap are folded out of alignment along their common fold line. The heel portion of each article A is thereby engaged in the resulting aperture and also received by the flaps to support and separating means.

Base panels 110 and 150 are brought into an overlapping relationship and connected together by locking tabs 164 and 166.

Handle tabs 44, 78 are folded about hand panels 30, 64 to further secure hand structure H and the carton as shown in Figure 2 of the drawings.

The present invention and its preferred embodiments relate to an article carrier which is shaped to provide satisfactory strength to hold the bottle securely but with a degree of flexibility so that load transferred to the handle is absorbed by the carrier. The shape of the blank minimises the amount of paperboard required. The carrier can be applied to an array of bottles by hand or automatic machinery. It is anticipated that the invention can be applied to a variety of carriers.

## Claims

1. An article carrier of the basket type adapted to accommodate a plurality of articles, for example, bottles, formed from a blank comprising, in sequence, a first side panel, a first end panel, a second side panel and a second end panel hinged one to the next, the carrier further comprising an internal partition structure (32, 34, 66, 68), handle means (30, 98, 64, 102) and article support and separating means (114, 132), said base comprises a pair of base panels (110, 150) each is associated with respective ones of said side walls (14, 18) and an intermediate panel (106, 140) is hinged between each base panel (110, 150) and the associated respective ones of side walls (14, 18), **characterised in that** said article support and separating means is struck from respective ones of intermediate panels (106, 146) and base panels (110, 150), said means comprising an aperture adapted to receive a portion of an article in use and a flap (114, 132) struck from and hingedly connected to an edge of said aperture, said flap is adapted to pivot inwardly of a carrier formed from the blank about its hinged connection.

2. An article carrier according to claim 1 wherein each of said article support and separating means comprises a pair of complementary hinged flaps (114, 132), each being hinged along opposite edges of the aperture.

3. An article carrier according to any preceding claim wherein said internal partition structure comprises a plurality of integral transverse partition panels (32, 34, 66, 68) wherein said transverse partition panels interconnect the handle means (30, 98, 64, 102) to each of the opposed side walls (14, 18) of the carrier, thereby creating a plurality of article receiving cells (C1 to C6) on both sides of the handle means.

4. An article carrier according to any preceding claim wherein a tab extends from each of said side panels into each of said apertures, each tab being interrupted by substantially vertical cut line (128) extending from a central portion of the lower edge of said tab and wherein the tab is disposed so as to abut and support a portion of an article (A) located at least in part in said aperture.

5. An article carrier according to claim 4 wherein each of said flaps (114, 132) includes an upper portion (116, 134) and a lower portion (118, 136) hinged so as to be foldable relative to one another and thereby allow the flap to bend and conform to the shape of an adjacent portion of an article and wherein the hinged connection (123) between said upper and lower flap portions is disposed above the base of an article (A) engaged therewith.

6. A blank for forming an article carrier of the basket type comprises in sequence a first side panel (14), a first end panel (16), a second side panel (18) and a second end panel (20), hinged one to the next, first and second base panels (110, 150) hinged to respective ones of said first and second intermediate panels (106, 146) which are in turn hingeably connected to said first and second side panels respectively and article support and separating means **characterised in that** said article support and separating means is struck from respective ones of intermediate panels (106, 146) and base panels (110, 150), said means comprising an aperture adapted to receive a portion of an article in use and a flap (114, 132) struck from and hingedly connected to an edge of said aperture, said flap is adapted to pivot inwardly of a carrier formed from the blank about its hinged connection.

7. A blank for forming an article carrier according to claim 6 further comprising first and second handle panels (30, 64) being connected to respective ones of said end panels (16, 20), and wherein a plurality of transverse partition panels (32, 34, 66, 68) interconnect the handle panels to each of the opposed side panels (14, 18) of the carrier, thereby creating a plurality of article receiving cells (C1 to C6) on both sides of the handle means when a carrier is formed from the blank.

8. A method of loading a basket carrier as claimed in any of claims 1 to 5, which method comprises the steps:
(i) setting up the carrier with its base panels open;
(ii) folding intermediate and base panels (106, 146, 110, 150) outwardly out of alignment with respective side panels (14, 18);
(iii) introducing articles (A) to the carton;
(iv) pivoting flaps (114, 132) of the article securing and supporting means about their hingeable connections (120, 122, 138, 140) into operative positions;
(v) folding the intermediate and base panels inwardly around the articles (A) such that the flaps (114, 132) receive and support respective articles;
(vi) interengaging said base panels (110, 150).

## Patentansprüche

1. Gegenstandsträger des Korbtyps, der angepasst ist, um eine Vielzahl von Gegenständen, z. B. Flaschen, aufzunehmen, und der aus einem Zuschnitt ausgebildet ist, welcher der Reihe nach eine erste Seitenwandfläche, eine erste Endwandfläche, eine zweite Seitenwandfläche und eine zweite Endwandfläche umfasst, die gelenkig eine mit der nächsten verbunden sind, wobei der Träger ferner eine innere Trennstruktur (32, 34, 66, 68), Griffmittel (30, 98, 64, 102) und Gegenstandsstütz- und Trennmittel (114, 132) umfasst, wobei die Basis ein Paar von Basiswandflächen (110, 150) umfasst, die jeweils mit den entsprechenden Seitenwänden (14, 18) verbunden sind, und wobei eine Zwischenwandfläche (106, 140) zwischen jeder Basiswandfläche (110, 150) und den dazugehörigen entsprechenden Seitenwänden (14, 18) gelenkig angebracht ist, **dadurch gekennzeichnet, dass** die Gegenstandsstütz- und Trennmittel aus den entsprechenden Zwischenwandflächen (106, 146) und Basiswandflächen (110, 150) ausgestanzt sind, wobei die Mittel eine Öffnung umfassen, die angepasst ist, um einen Abschnitt eines Gegenstands in Gebrauch aufzunehmen, sowie eine Klappe (114, 132), die aus einer Kante der Öffnung ausgestanzt und damit gelenkig verbunden ist, wobei die Klappe angepasst ist, um in das Innere eines aus dem Zuschnitt ausgebildeten Trägers um dessen gelenkige Verbindung zu schwenken.

2. Gegenstandsträger nach Anspruch 1, in welchem jedes der Gegenstandsstützund Trennmittel ein Paar von komplementären schwenkbaren Klappen (114, 132) umfasst, die jeweils entlang gegenüberliegender Kanten der Öffnung gelenkig angebracht sind.

3. Gegenstandsträger nach einem der vorstehenden Ansprüche, in welchem die innere Trennstruktur eine Vielzahl von integralen quer verlaufenden Trennwandflächen (32, 34, 66, 68) umfasst, wobei die quer verlaufenden Trennwandflächen die Griffmittel (30, 98, 64, 102) mit jeder der gegenüberliegenden Seitenwände (14, 18) des Trägers verbinden, wodurch eine Vielzahl von Gegenstandsaufnahmezellen (C1 bis C6) auf beiden Seiten der Griffmittel erzeugt wird.

4. Gegenstandsträger nach einem der vorstehenden Ansprüche, in welchem sich eine Lasche von jeder der Seitenwandflächen in jede der Öffnungen erstreckt, wobei jede Lasche durch eine im Wesentlichen vertikale Stanzlinie (128) unterbrochen wird, die sich von einem zentralen Abschnitt der unteren Kante der Lasche erstreckt, und wobei die Lasche derart angeordnet ist, dass sie an einen Abschnitt eines wenigstens teilweise in der Öffnung angeordneten Gegenstands (A) anstößt und diesen hält.

5. Gegenstandsträger nach Anspruch 4, in welchem jede der Klappen (114, 132) einen oberen Abschnitt (116, 134) und einen unteren Abschnitt (118, 136) einschließt, die derart gelenkig angebracht sind, dass sie relativ zueinander faltbar sind und dadurch der Klappe erlauben, sich zu verbiegen und sich an die Form eines angrenzenden Abschnitts eines Gegenstands anzupassen, und in welchem die gelenkige Verbindung (123) zwischen dem oberen und unteren Klappenabschnitt oberhalb der Basis eines Gegenstands (A) damit in Eingriff genommen angeordnet ist.

6. Zuschnitt zum Ausbilden eines Gegenstandsträgers des Korbtyps, welcher der Reihe nach eine erste Seitenwandfläche (14), eine erste Endwandfläche (16), eine zweite Seitenwandfläche (18) und eine zweite Endwandfläche (20) umfasst, die eine mit der nächsten gelenkig verbunden sind, eine erste und eine zweite Basiswandfläche (110, 150), die mit den entsprechenden ersten und zweiten Zwischenwandflächen (106, 146) gelenkig verbunden sind, die wiederum mit der ersten bzw. zweiten Seitenwandfläche gelenkig verbunden sind, sowie Gegenstandsstütz- und Trennmittel, **dadurch gekennzeichnet, dass** die Gegenstandsstütz- und Trennmittel aus den entsprechenden Zwischenwandflächen (106, 146) und Basiswandflächen (110, 150) ausgestanzt sind, wobei die Mittel eine Öffnung umfassen, die angepasst ist, um einen Abschnitt eines Gegenstands in Gebrauch aufzunehmen, sowie eine Klappe (114, 132), die aus einer Kante der Öffnung ausgestanzt und damit gelenkig verbunden ist, wobei die Klappe angepasst ist, um in das Innere eines aus dem Zuschnitt ausgebildeten Trägers um dessen gelenkige Verbindung zu schwenken.

7. Zuschnitt zum Ausbilden eines Gegenstandsträgers nach Anspruch 6, welcher ferner erste und zweite Griffwandflächen (30, 64) umfasst, die mit den entsprechenden Endwandflächen (16, 20) verbunden sind, und in welchem eine Vielzahl von quer verlaufenden Trennwandflächen (32, 34, 66, 68) die Griffwandflächen mit jeder der gegenüberliegenden Seitenwandflächen (14, 18) des Trägers verbindet, wodurch eine Vielzahl von Gegenstandsaufnahmezellen (C1 bis C6) auf beiden Seiten der Griffmittel erzeugt wird, wenn ein Träger aus dem Zuschnitt ausgebildet wird.

8. Verfahren zum Beladen eines Trägers des Korbtyps nach einem der Ansprüche 1 bis 5, wobei das Verfahren die folgenden Schritte umfasst:
(i) Aufrichten des Trägers mit geöffneten Basiswandflächen;
(ii) Falten der Zwischen- und Basiswandflächen (106, 146, 110, 150) nach außen aus der Ausrichtung mit den entsprechenden Seitenwandflächen (14, 18);
(iii) Einführen von Gegenständen (A) in die Schachtel;
(iv) Schwenken der Klappen (114, 132) der Gegenstandsbefestigungs- und Stützmittel um ihre gelenkigen Verbindungen (120, 122, 138, 140) in funktionsfähige Positionen;
(v) Falten der Zwischen- und Basiswandflächen nach innen um die Gegenstände (A), derart, dass die Klappen (114, 132) entsprechende Gegenstände aufnehmen und halten;
(vi) Ineinanderschieben der Basiswandflächen (110, 150).

## Revendications

1. Dispositif de transport d'objet de type panier adapté pour loger une pluralité d'objets, par exemple, des bouteilles, formé d'une ébauche comprenant, par ordre, un premier panneau latéral, un premier panneau d'extrémité, un deuxième panneau latéral et un deuxième panneau d'extrémité, articulé le précédent sur le suivant, le dispositif de transport comprenant en outre une structure de séparation interne (32, 34, 66, 68), des moyens de poignée (30, 98, 64, 102) et des moyens de support et de séparation d'objet (114, 132), ladite base comprenant une paire de panneaux de base (110, 150), dont chacun est associé avec les panneaux respectifs desdites parois latérales (14, 18) et un panneau intermédiaire (106, 140) est articulé entre chaque panneau de base (110, 150) et les panneaux respectifs associés des parois latérales (14, 18), **caractérisé en ce que** lesdits moyens de support et de séparation d'objet sont touchés par des moyens respectifs de panneaux intermédiaires (106, 146) et des panneaux de base (110, 150), lesdits moyens comprenant une ouverture adaptée pour recevoir une partie d'un objet en cours d'utilisation et un volet (114, 132) touché par et relié par articulation à un bord de ladite ouverture, ledit volet étant adapté pour pivoter vers l'intérieur d'un dispositif de transport formé par l'ébauche autour de sa liaison par articulation.

2. Dispositif de transport d'objet selon la revendication 1, dans lequel chacun desdits moyens de support et de séparation d'objet comprend une paire de volets articulés complémentaires (114, 132), chacun d'entre eux étant articulé le long de bords opposés de l'ouverture.

3. Dispositif de transport d'objet selon l'une quelconque des revendications précédentes, dans lequel ladite structure de séparation interne comprend une pluralité de panneaux de séparation transversaux intégraux (32, 34, 66, 68), dans laquelle lesdits panneaux de séparation transversaux relient les moyens de poignée (30, 98, 64, 102) à chacune des parois latérales opposées (14, 18) du dispositif de transport, créant par ce moyen une pluralité de cellules de réception d'objet (C 1 à C 6) des deux côtés des moyens de poignée.

4. Dispositif de transport d'objet selon l'une quelconque des revendications précédentes, dans lequel une patte s'étend depuis chacun desdits panneaux latéraux dans chacune desdites ouvertures, chaque patte étant interrompue par une ligne de coupe sensiblement verticale (128) s'étendant depuis une partie centrale du bord inférieur de ladite patte et dans laquelle la patte est disposée de façon à buter contre et à supporter une partie d'un objet (A) situé au moins en partie dans ladite ouverture.

5. Dispositif de transport d'objet selon la revendication 4, dans lequel chacun desdits volets (114, 132) comporte une partie supérieure (116, 134) et une partie inférieure (118, 136) articulée de façon à être pliable l'une par rapport à l'autre et permettant par ce moyen au volet de fléchir et de s'adapter à la forme d'une partie adjacente d'un objet et dans lequel la liaison articulée (123) entre lesdites parties de volet supérieure et inférieure est disposée au-dessus de la base d'un objet (A) en prise avec celle-ci.

6. Ebauche destinée à former un dispositif de transport d'objet de type panier comprenant par ordre un premier panneau latéral (14), un premier panneau d'extrémité (16), un deuxième panneau latéral (18) et un deuxième panneau d'extrémité (20), le précédent articulé sur le suivant, les premier et deuxième panneaux de base (110, 150) articulés sur les panneaux respectifs desdits premier et deuxième panneaux intermédiaires (106, 146), qui sont à leur tour reliés par articulation aux dits premier et deuxième panneaux latéraux respectivement et des moyens de support et de séparation d'objet, **caractérisée en ce que** lesdits moyens de support et de séparation d'objet sont touchés par des moyens respectifs de panneaux intermédiaires (106, 146) et des panneaux de base (110, 150), lesdits moyens comprenant une ouverture adaptée pour recevoir une partie d'un objet en cours d'utilisation et un volet (114, 132), touché par et relié par articulation à un bord de ladite ouverture, ledit volet étant adapté pour pivoter vers l'intérieur d'un dispositif de transport formé par l'ébauche autour de sa liaison par articulation.

7. Ebauche destinée à former un dispositif de transport d'objet selon la revendication 6 comprenant en outre des premier et deuxième panneaux à poignée (30, 64) reliés à des panneaux respectifs desdits panneaux d'extrémité (16, 20) et dans laquelle une pluralité de panneaux de séparation transversaux (32, 34, 66, 68) relie les panneaux à poignée à chacun des panneaux latéraux opposés (14, 18) du dispositif de transport, créant par ce moyen une pluralité de cellules de réception d'objet (C 1 à C 6) des deux côtés du moyen de poignée quand un dispositif de transport est formé à partir de l'ébauche.

8. Procédé de chargement d'un dispositif de transport de type panier selon l'une quelconque des revendications 1 à 5, procédé comprenant les étapes consistant à :
(i) dresser le dispositif de transport avec ses panneaux de base ouverts ;
(ii) plier les panneaux intermédiaires et de base (106, 146, 110, 150) vers l'extérieur non alignés avec les panneaux latéraux respectifs (14, 18) ;
(iii) introduire des objets (A) sur le carton ;
(iv) faire pivoter les volets (114, 132) des moyens de fixation et de support d'objet autour de leurs liaisons articulables (120, 122, 138, 140) dans des positions actives ;
(v) plier les panneaux intermédiaires et de base vers l'intérieur autour des objets (A) de sorte que les volets (114, 132) reçoivent et supportent des objets respectifs ;
(vi) mettre en prise entre eux lesdits panneaux de base (110, 150).
